# EUROPEAN PATENT APPLICATION

(11) **EP 1 985 232 A1**
(43) Date of publication of application: **29.10.2008**
(21) Application number: 06843596.5
(22) Date of filing: 28.12.2006
(51) Int. Cl.: A61B 5/0404, A61B 5/0408, A61B 5/0492

(54) **PORTABLE ELECTROCARDIOGRAPH**

(30) Priority: 10.02.2006 JP 2006034163
(71) Applicant: Omron Healthcare Co., Ltd., Kyoto 615-0084 (JP)
(72) Inventor: UMEDA, Masahiro, Kyoto-shi, Kyoto 615-0084 (JP); YAMAMOTO, Norihito, Kyoto-shi, Kyoto 615-0084 (JP); SAKODA, Yusaku, Kyoto-shi, Kyoto 615-0084 (JP)
(74) Representative: Wilhelms · Kilian & Partner Patentanwälte
(86) International application number: PCT/JP2006/326218
(87) International publication number: WO 2007/091379

(57) **Abstract**

A portable electrocardiograph (1A) includes a body unit (100) arranged with a negative electrode and a detachable unit (110) arranged with a positive electrode, where the detachable unit (110) is detachably attached to the body unit (100). The negative electrode and the positive electrode are both exposed in a state where the detachable unit (110) is united to the body unit (100). In the portable electrocardiograph (1A), an electrocardiographic waveform can be measured both in a state where the detachable unit (110) is united to the body unit (100) and in a state where the detachable unit (110) is separated from the body unit (100). According to such a configuration, it is possible to obtain the portable electrocardiograph in which a degree of freedom in measuring position is enhanced such that the electrocardiographic waveform can be measured by selecting a specific measuring position from a plurality of measuring positions according to a situation.

## Description

### TECHNICAL FIELD

The present invention relates to a portable electrocardiograph that can be carried around and that can easily measure an electrocardiographic waveform.

### BACKGROUND ART

An electrocardiogram of a patient is generally used to diagnose arrhythmia, and ischemic heart disease such as cardiac angina and myocardial infarction. Various configurations have been proposed for the electrocardiograph used to obtain the electrocardiogram. One of those is a portable electrocardiograph that can be carried around, and that measures and stores the electrocardiographic waveform when the examinee himself/herself brings the electrode into contact with the body when a certain symptom to be measured occurs. This portable electrocardiograph is classified as an event-type electrocardiograph, and is distinguished from a halter-type electrocardiograph in which the electrode is attached to the body of the patient beforehand, and the electrocardiographic waveform is successively measured and recorded while going about his/her daily life.

Documents disclosing such a portable electrocardiograph include Japanese Patent Publication Laid-Open No. 2005-40187 (Patent Document 1), Japanese Patent Publication Laid-Open No. 2005-185756 (Patent Document 2), and the like. The portable electrocardiograph disclosed in Patent Document 1 has an electrode, which is to be mainly brought into contact with the skin at the fifth intercostal space in the anterior axillary line of the abdominal, arranged on an end face positioned at one end in the longitudinal direction of a device body having a flat and elongate substantially rectangular solid shape, an electrode, which is to be mainly brought into contact with the right hand that grips a grip part, arranged on the grip part including an end face positioned on the side opposite to the former one end, where the electrocardiographic waveform is obtained by measuring a potential difference generated between the pair of electrodes. Furthermore, the portable electrocardiograph disclosed in Patent Document 2 obtains the electrocardiographic waveform by bringing the electrode arranged on an outer surface of the device body and the electrode pulled out to the outside from the device body by a connection cable mainly into contact with the skin at the fifth intercostal space in the anterior axillary line of the abdominal and the right hand, respectively, and measuring the potential difference generated between the pair of electrodes.
[Patent Document 1] Japanese Patent Publication Laid-Open No. 2005-40187
[Patent Document 2] Japanese Patent Publication Laid-Open No. 2005-185756

### DISCLOSURE OF THE INVENTION

### PROBLEMS TO BE SOLVED BY THE INVENTION

The portable electrocardiograph measures the electrocardiographic waveform when the examinee himself/herself brings the electrode into contact with the body when symptoms to be measured such as palpitation and shortness of breath occur. Therefore, it is important that consideration is made to enable the measuring position to be taken as quickly as possible. The portable electrocardiographs disclosed in Patent Documents 1 and 2 are respectively reviewed so as to be user-friendly, but the degree of freedom in the selection of the measuring position is not very high since the measuring position is more or less uniquely determined in the respective portable electrocardiograph. Therefore, improvements still can be made from such an aspect, and it is important to enhance the degree of freedom in the measuring position so that a specific measuring position can be selected from a plurality of measuring positions according to a situation to measure the electrocardiographic waveform.

In view of solving the above problems, it is an object of the present invention to provide a portable electrocardiograph in which the degree of freedom in the measuring position is enhanced so that a specific measuring position can be selected from a plurality of measuring positions according to a situation to measure the electrocardiographic waveform.

### MEANS FOR SOLVING THE PROBLEMS

A portable electrocardiograph based on a first aspect of the present invention includes a first unit arranged with a first electrode and a second unit arranged with a second electrode, and obtains an electrocardiographic waveform by measuring a potential difference generated between the first and the second electrodes brought into contact with a body surface. The second unit is detachably attached to the first unit. The first electrode and the second electrode are both exposed in a state where the second unit is united to the first unit. The measurement of the potential difference for obtaining the electrocardiographic waveform is made between the first and the second electrodes both in a state where the second unit is united to the first unit and in a state where the second unit is separated from the first unit.

According to such a configuration, the electrocardiographic waveform can be obtained by measuring the potential difference generated between the first and the second electrodes in at least two states including the state where the second unit is united to the first unit and the state where the second unit is separated from the first unit. Therefore, the examinee himself/herself can select a specific measuring position from a plurality of measuring positions according to a situation, and the electrocardiographic waveform can be measured quickly and easily when a certain symptom to be measured occurs. A portable electrocardiograph excelling in handleability is thereby obtained.

A portable electrocardiograph based on a second aspect of the present invention includes a first unit arranged with a first electrode, and a second unit arranged with a second electrode and a third electrode, and obtains an electrocardiographic waveform by measuring a potential difference generated between the first and the second electrodes brought into contact with a body surface or between the third and the second electrodes brought into contact with a body surface. The second unit is detachably attached to the first unit. The first electrode and the second electrode are exposed in a state where the second unit is united to the first unit and the third electrode is covered by the first unit in the state where the second unit is united to the first unit. The first electrode, the second electrode, and the third electrode are all exposed in a state where the second unit is separated from the first unit. The measurement of the potential difference for obtaining the electrocardiographic waveform is made between the first and the second electrodes in a state where the second unit is united to the first unit, and is made between the third and the second electrodes in a state where the second unit is separated from the first unit.

According to such a configuration, the electrocardiographic waveform can be obtained by measuring the potential difference generated between the first and the second electrodes or between the third and the second electrodes in at least two states including the state where the second unit is united to the first unit and the state where the second unit is separated from the first unit. Therefore, the examinee himself/herself can select a specific measuring position from a plurality of measuring positions according to a situation, and the electrocardiographic waveform can be measured quickly and easily when a certain symptom to be measured occurs. A portable electrocardiograph excelling in handleability is thereby obtained.

In the portable electrocardiograph based on the second aspect of the present invention, a switching unit is preferably arranged for switching between the first electrode and the third electrode to be used to obtain the electrocardiographic waveform. In this case, the switching unit is preferably configured to perform a switching operation in conjunction with at least one of an operation of separating the second unit from the first unit and an operation of uniting the second unit to the first unit.

According to such a configuration, the electrode not used in the measurement of the electrocardiographic waveform of the first or the third electrode can be separated from the processing circuit. Therefore, superimposition etc. of noise can be prevented and accurate measurement can be made. The switching operation of the switching unit is cooperatively carried out with the detachment operation of the second unit from the first unit or the attachment operation of the second unit to the first unit performed by the examinee, so that a troublesome switching task is not forced on the examinee and the switching of the electrode is reliably carried out. A portable electrocardiograph excelling in handleability is thereby obtained.

In the portable electrocardiograph based on the first aspect and the second aspect of the present invention, a measurement button for starting a measurement of the electrocardiographic waveform is preferably arranged on both the first unit and the second unit.

According to such a configuration, the examinee himself/herself can selectively operate the measurement button that is easy to operate from the measurement button arranged in the first unit and the measurement button arranged in the second unit, and thus the handleability greatly enhances both in the state where the second unit is united to the first unit and in the state where the second unit is separated from the first unit.

In the portable electrocardiograph based on the first aspect and the second aspect of the present invention, a winding mechanism for winding and accommodating the connection cable is preferably arranged either in the first unit or in the second unit when the first unit and the second unit are connected by a connection cable.

Therefore, the handleability of the connection cable in detaching the second unit from the first unit and in attaching the second unit to the first unit significantly enhances by arranging a winding mechanism of the connection cable either in the first unit or in the second unit as in the configuration described above when wire connecting the first unit and the second unit.

In the portable electrocardiograph based on the first aspect and the second aspect of the present invention, a device body including the first unit and the second unit preferably has a substantially rectangular solid shape in the state where the second unit is united to the first unit, in which case, the second electrode is preferably arranged on one end face in a longitudinal direction of the device body. In this case, the first electrode is preferably an electrode to be brought into contact with a right hand; and the second electrode is preferably an electrode to be brought into contact with a chest region.

According to such a configuration, the first electrode and the second electrode are respectively positioned on opposite sides in the longitudinal direction of the device body in the state where the second unit is united to the first unit, and thus the first and the second electrodes are respectively easily brought into contact with a predetermined measuring site when the examinee takes the measuring position.

In the portable electrocardiograph based on the first aspect and the second aspect of the present invention, a display unit for displaying a measurement result is preferably arranged in the first unit.

According to such a configuration, the visibility of the display unit enhances when the examinee selects, in particular, the measuring position with the second unit separated from the first unit. Therefore, if such a measuring position is selected, the display unit can be easily checked during the measurement, and failure in data acquisition due to operation mistake by the examinee can be prevented in advance.

In the portable electrocardiograph based on the first aspect and the second aspect of the present invention, an attachment mechanism enabling attachment to a living body is preferably arranged in the first unit. In particular, the attachment mechanism is preferably a wrapping member to be wrapped around a wrist.

According to such a configuration, a portable electrocardiograph excelling in portability is obtained, whereby the measuring position can be quickly taken when a certain symptom to be measured occurs by wearing the portable electrocardiograph.

### EFFECT OF THE INVENTION

According to the present invention, a portable electrocardiograph in which degree of freedom in the measuring position is enhanced such that the electrocardiographic waveform can be measured by selecting a specific measuring position from a plurality of measuring positions according to a situation is obtained. Therefore, a user-friendly portable electrocardiograph capable of rapidly and easily measuring the electrocardiographic waveform when a certain symptom to be measured occurs is obtained.

### BRIEF DESCRIPTION OF THE DRAWINGS

Fig. 1 is a perspective view showing a state where a detachable unit is united to a body unit in a portable electrocardiograph according to a first embodiment of the present invention.
Fig. 2 is a perspective view showing the state where the detachable unit is united to the body unit in the portable electrocardiograph according to the first embodiment of the present invention.
Fig. 3 is a front view showing the state where the detachable unit is united to the body unit in the portable electrocardiograph according to the first embodiment of the present invention.
Fig. 4 is a top view showing the state where the detachable unit is united to the body unit in the portable electrocardiograph according to the first embodiment of the present invention.
Fig. 5 is a bottom view showing the state where the detachable unit is united to the body unit in the portable electrocardiograph according to the first embodiment of the present invention.
Fig. 6 is a right side view showing the state where the detachable unit is united to the body unit in the portable electrocardiograph according to the first embodiment of the present invention.
Fig. 7 is a left side view showing the state where the detachable unit is united to the body unit in the portable electrocardiograph according to the first embodiment of the present invention.
Fig. 8 is a front view showing a state where the detachable unit is separated from the body unit in the portable electrocardiograph according to the first embodiment of the present invention.
Fig. 9 is a functional block diagram of the portable electrocardiograph according to the first embodiment of the present invention.
Fig. 10 is a flowchart showing a flow of process performed in the portable electrocardiograph according to the first embodiment of the present invention.
Fig. 11 is a view showing a display example of a measurement result in the portable electrocardiograph according to the first embodiment of the present invention.
Fig. 12 is a view showing another display example of the measurement result in the portable electrocardiograph according to the first embodiment of the present invention.
Fig. 13 is a view showing a measuring position to be taken by an examinee when measuring an electrocardiographic waveform with the detachable unit united to the body unit in the portable electrocardiograph according to the first embodiment of the present invention.
Fig. 14 is a view showing the measuring position to be taken by the examinee when measuring the electrocardiographic waveform with the detachable unit united to the body unit in the portable electrocardiograph according to the first embodiment of the present invention.
Fig. 15 is a view showing a gripping state by a right hand of the examinee when the examinee takes the measuring position shown in Fig. 13 and Fig. 14.
Fig. 16 is a view showing a measuring position to be taken by an examinee when measuring the electrocardiographic waveform in a state where the detachable unit is separated from the body unit in the portable electrocardiograph according to the first embodiment of the present invention.
Fig. 17 is a view showing the measuring position to be taken by the examinee when measuring the electrocardiographic waveform in the state where the detachable unit is separated from the body unit in the portable electrocardiograph according to the first embodiment of the present invention.
Fig. 18 is a view showing a gripping state by a left hand of the examinee when the examinee takes the measuring position shown in Fig. 16 and Fig. 17.
Fig. 19 is a perspective view showing a state where a detachable unit is united to a body unit in a portable electrocardiograph according to a second embodiment of the present invention.
Fig. 20 is a perspective view showing the state where the detachable unit is united to the body unit in the portable electrocardiograph according to the second embodiment of the present invention.
Fig. 21 is a perspective view of the detachable unit in a state where the body unit is separated from the detachable unit in the portable electrocardiograph according to the second embodiment of the present invention.
Fig. 22 is a front view showing the state where the detachable unit is separated from the body unit in the portable electrocardiograph according to the second embodiment.
Fig. 23 is a functional block diagram of the portable electrocardiograph according to the second embodiment of the present invention.
Fig. 24 is a view showing a measuring position to be taken by an examinee when measuring an electrocardiographic waveform with the detachable unit separated from the body unit in the portable electrocardiograph according to the second embodiment of the present invention.
Fig. 25 is a view showing the measuring position to be taken by the examinee when measuring the electrocardiographic waveform with the detachable unit separated from the body unit in the portable electrocardiograph according to the second embodiment of the present invention.
Fig. 26 is a view showing a gripping state by a right hand of the examinee when the examinee takes the measuring position shown in Fig. 24 and Fig. 25.
Fig. 27 is a perspective view showing a state where a detachable unit is united to a body unit in a portable electrocardiograph according to a third embodiment of the present invention.
Fig. 28 is a perspective view showing a state where the detachable unit is separated from the body unit in the portable electrocardiograph according to the third embodiment of the present invention.
Fig. 29 is a perspective view showing a state where a detachable unit is united to a body unit in a portable electrocardiograph according to a fourth embodiment of the present invention.
Fig. 30 is a rear view showing the state where the detachable unit is united to the body unit in the portable electrocardiograph according to the fourth embodiment of the present invention.
Fig. 31 is a perspective view showing a state where the detachable unit is separated from the body unit in the portable electrocardiograph according to the fourth embodiment of the present invention.

### DESCRIPTION OF SYMBOLS

- 1A to 1D: portable electrocardiograph
- 100: body unit
- 101: front face
- 102: rear face
- 102a to 102d: opening
- 103: upper face
- 104: lower face
- 105: right side face
- 105a: concave part
- 106: left side face
- 107A, 107B: release button
- 107a, 107b: engagement projection
- 108: accommodating section
- 109: cutout part
- 110: detachable unit
- 111: front face
- 112: rear face
- 113: upper face
- 113a: engagement concave part
- 114: lower face
- 114a: engagement concave part
- 115: right side face
- 116: left side face
- 118: alignment mark
- 120: electrode unit
- 121, 123: negative electrode
- 122: positive electrode
- 124,: 124A, 124B indifferent electrode
- 126: detachment detection sensor
- 127A, 127B: switch
- 130: open/close cover
- 132: external storage medium
- 140: operation unit
- 141: power button
- 142, 142A, 142B: measurement button
- 143: menu button
- 144: determination button
- 145: left scroll button
- 146: right scroll button
- 148: display unit
- 150: processing circuit
- 151: amplifier circuit
- 152: filter circuit
- 153: A/D converter
- 154: CPU
- 155: memory
- 156: timer
- 160: power
- 170: connection cable
- 180: reel
- 190: band
- 191: opening
- 200: examinee
- 210: right hand
- 211: thumb
- 212: forefinger
- 220: forearm
- 230: upper arm
- 240: right shoulder
- 250: chest region
- 260: left hand
- 261: thumb
- 262: forefinger
- 300: desk

### BEST MODE FOR CARRYING OUT THE INVENTION

Embodiments of the present invention will now be described in detail with reference to the drawings.

### (First embodiment)

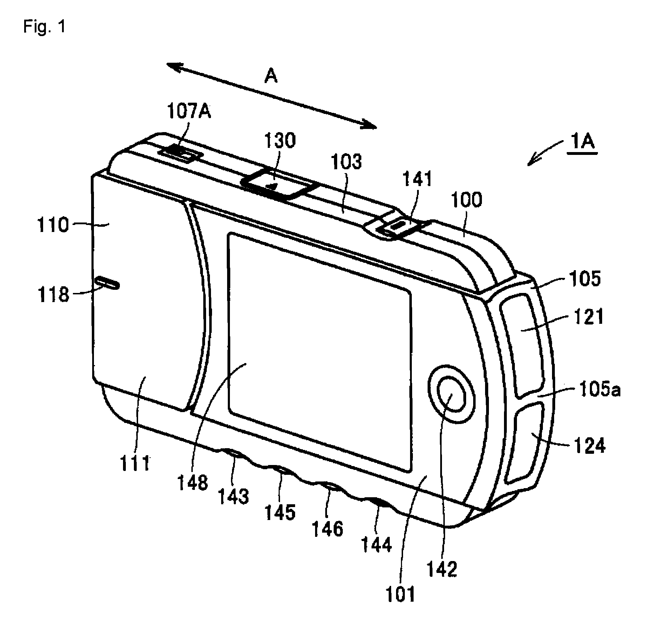

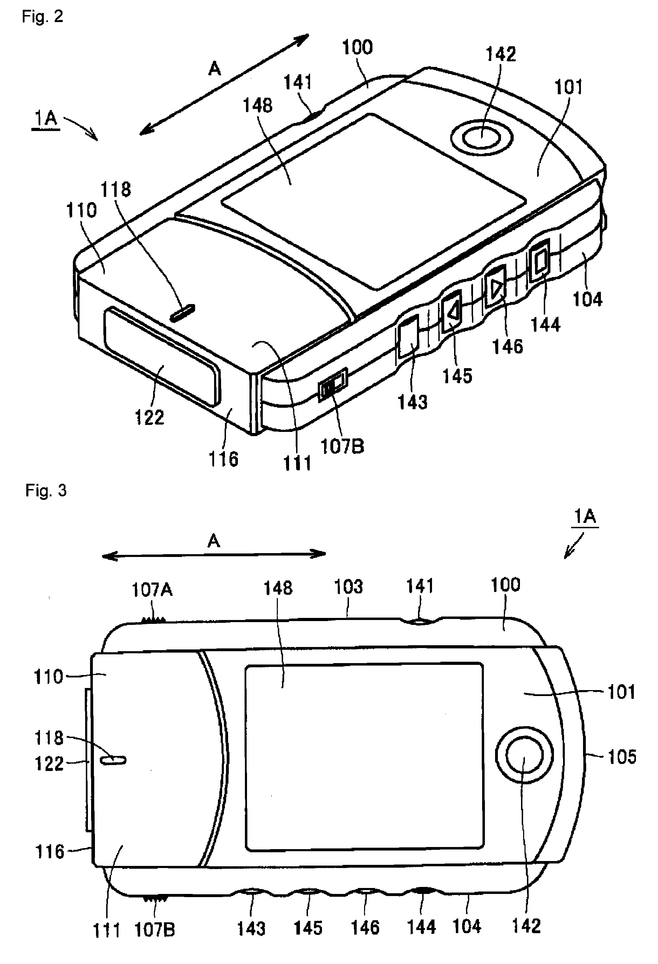

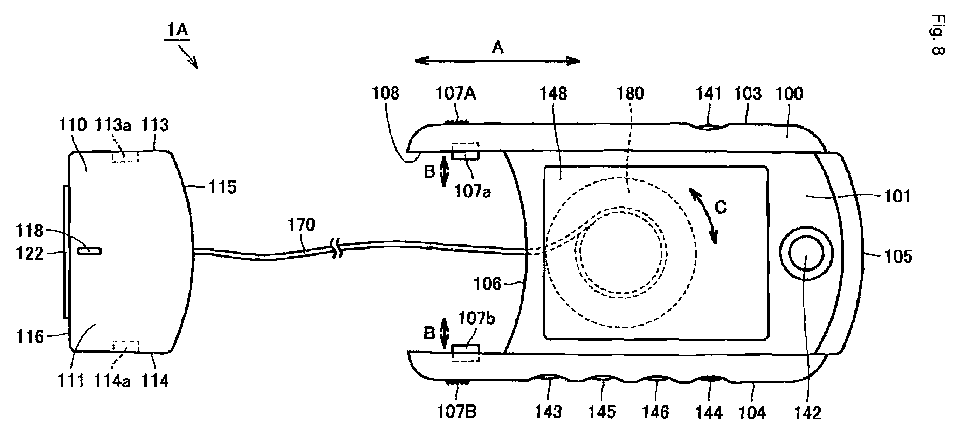

Fig. 1 and Fig. 2 are perspective views showing a state where a body unit and a detachable unit are united to each other in a portable electrocardiograph according to a first embodiment of the present invention. Fig. 3 to Fig. 7 are a front view, a top view, a bottom view, a right side view, and a left side view of the state where the body unit and the detachable unit are united to each other in the portable electrocardiograph of the present embodiment. Fig. 8 is a front view showing a state where the detachable unit is detached from the body unit in the portable electrocardiograph of the present embodiment.

First, an outer appearance structure of the portable electrocardiograph with the detachable unit united with the body unit will be described. As shown in Fig. 1 to Fig. 7, the portable electrocardiograph 1A according to the present embodiment is miniaturized and lightened to a size and weight capable of being held by one hand so as to excel in handleability. The device body of the portable electrocardiograph 1A includes a body unit 100 serving as a first unit, and a detachable unit 110 serving as a second unit. The detachable unit 110 is detachably attached to the body unit 100.

The body unit 100 has an outer shape of a flat and elongate substantially rectangular solid shape extending in a direction of an arrow A in the figure, where a display unit, an operation unit, an electrode, and the like are disposed on the outer surface (front face 101, rear face 102, upper face 103, lower face 104, right side face 105, and left side face 106 (see Fig. 8 for left side face 106)). The detachable unit 110 has an outer shape of a flat substantially rectangular solid shape, where an electrode and the like are disposed on the outer surface (front face 111, rear face 112, upper face 113, lower face 114, right side face 115, and left side face 116 (see Fig. 8 for upper face 113, lower face 114, and right side face 115).

As shown in Fig. 1 to Fig. 7, when the detachable unit 110 is united with the body unit 100, the detachable unit 110 is accommodated in an accommodating section 108 (see Fig. 8) at the left side face 106 of the body unit 100, so that the outer shape of the device body of the portable electrocardiograph 1A as a whole becomes a flat substantially rectangular shape extending long in a direction of an arrow A in the figure. In this state, the detachable unit 110 is engaged to the body unit 100 by an engagement mechanism to be hereinafter described, so that the left side face 106 of the body unit 100 and the right side face 115 of the detachable unit 110 face each other.

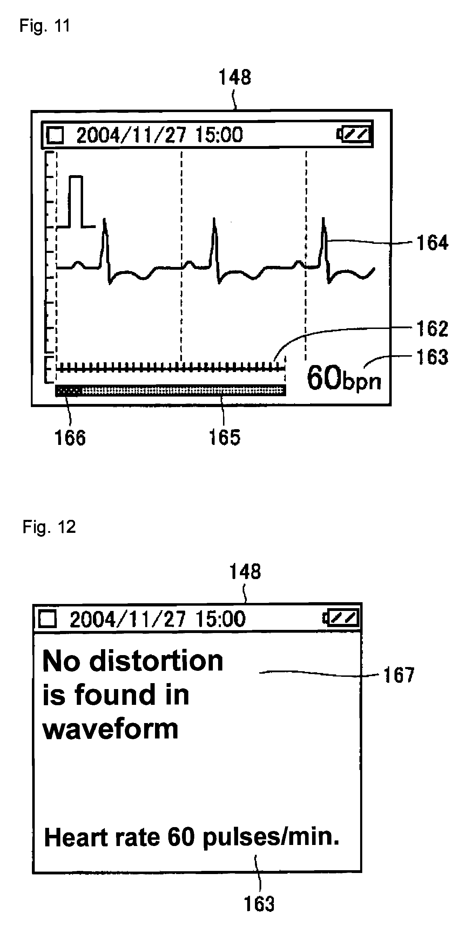

As shown in Fig. 1 to Fig. 3, a measurement button 142 is arranged at a predetermined position of the front face 101 of the body unit 100. The measurement button 142 is an operation button for starting the measurement of the electrocardiographic waveform. Furthermore, a display unit 148 is arranged at a predetermined position of the front face 101 of the body unit 100. The display unit 148 is configured by a liquid crystal display, and the like, and is a site for displaying measurement result etc. The measurement result is displayed as electrocardiographic waveform and numerical data, as shown in Fig. 11 and Fig. 12. An alignment mark 118 that becomes a mark when pressing a positive electrode 122, to be hereinafter described, against the body surface is arranged at an end closer to the left side face 116 of the front face 111 of the detachable unit 110.

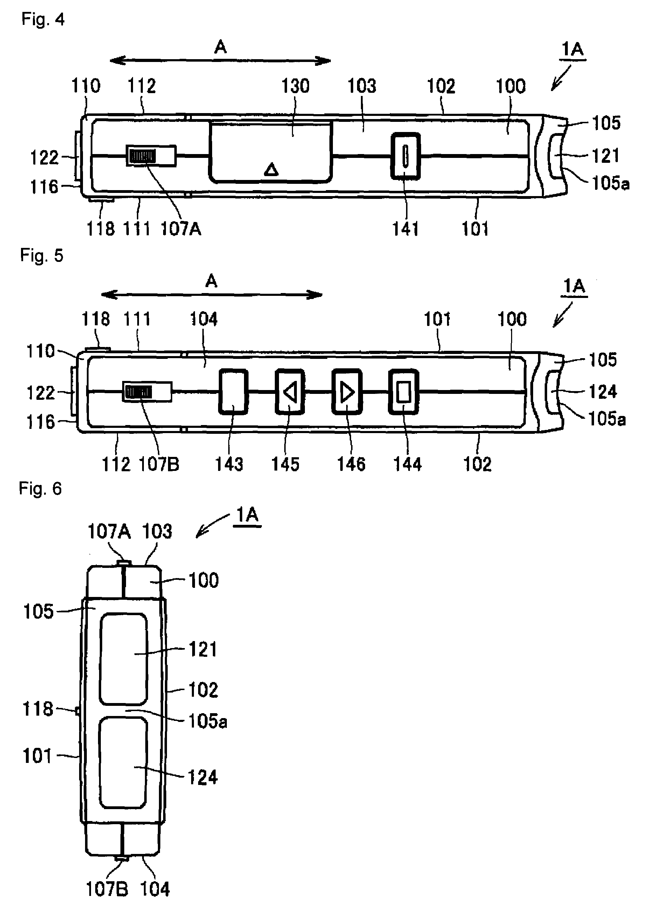

As shown in Fig. 1 and Fig. 4, a power button 141 is disposed at a predetermined position of the upper face 103 of the body unit 100. The power button 141 is an operation button for operating ON/OFF of the portable electrocardiograph 1A. An open/close cover 130 is arranged at a predetermined position of the upper face 103 of the body unit 100. The open/close cover 130 is arranged to cover a slot, to which an external storage medium is inserted, in the closed state, and is attached to the body unit 100 in a freely opening/closing manner. A release button 107A is arranged on the upper face 103 of the body unit 100 at the portion adjacent to the accommodating section 108. The release button 107A is a button that is operated when detaching the detachable unit 110 from the body unit 100.

As shown in Fig. 2 and Fig. 5, various operation buttons are disposed at a predetermined position of the lower face 104 of the body unit 100. In the illustrated portable electrocardiograph 1A, a menu button 143, a determination button 144, a left scroll button 145, and right scroll button 146 are disposed. The menu button 143 is an operation button for instructing display of a menu on the display unit 148 in the portable electrocardiograph 1A, and the determination button 144 is an operation button for executing a setting operation associated with the information displayed on the display unit 148.
The left scroll button 145 and the right scroll button 146 are operation buttons for displaying graphs of the measurement result, guide information and the like displayed on the display unit 148 by scrolling. Similar to the upper face 103 described above, a release button 107B is arranged on the lower face 104 of the body unit 100 at the portion adjacent to the accommodating section 108. The release button 107B is a button that is operated when detaching the detachable unit 110 from the body unit 100.

As shown in Fig. 1 and Fig. 6, a negative electrode 121, which is a first electrode, and an indifferent electrode (neutral electrode) 124 for obtaining a potential that becomes the reference of potential change of the body are disposed on the right side face 105 of the body unit 100 positioned at one end in the longitudinal direction (direction of arrow A in the figure) of the device body. The right side face 105 has a smoothly curved shape so that the forefinger of the right hand of the examinee fits thereto when the examinee takes a measuring position to be hereinafter described. Furthermore, a concave part 105a extending in the up and down direction is formed in the right side face 105. The concave part 105a has a shape for receiving the forefinger of the right hand of the examinee.

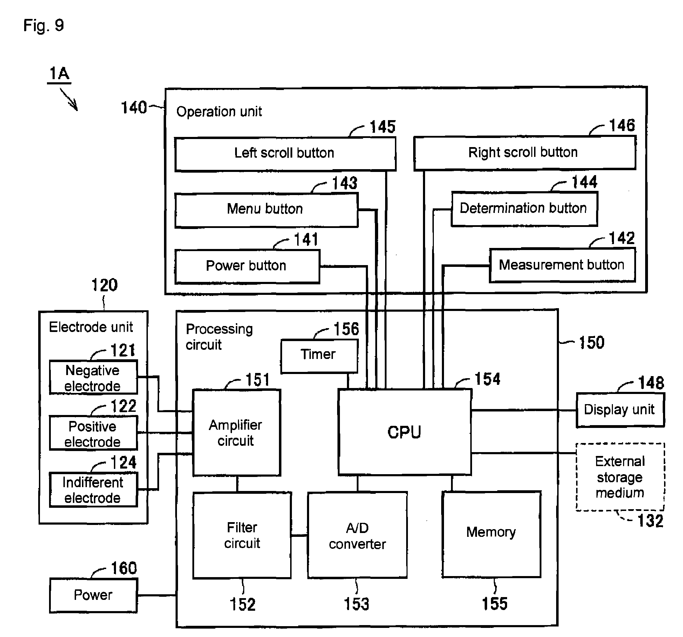

The negative electrode 121 and the indifferent electrode 124 described above are formed by a conductive member, and are electrically connected to a processing circuit 150 (see Fig. 9) arranged in the body unit 100. The negative electrode 121 and the indifferent electrode 124 are disposed so that the surfaces thereof are exposed at the outer surface of the body unit 100 in the concave part 105a formed in the right side face 105 of the body unit 100. The negative electrode 121 is positioned closer to the upper face 103 of the right side face 105, and the indifferent electrode 124 is positioned closer to the lower face 104 of the right side face 105.

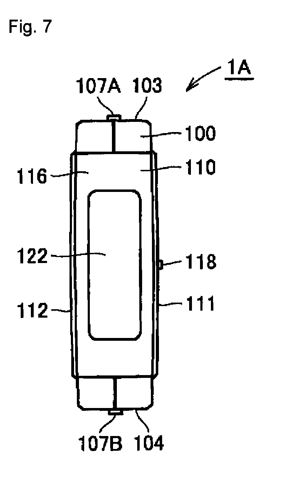

As shown in Fig. 2 and Fig. 7, the positive electrode 122, which is a second electrode, is arranged on the left side face 116 of the detachable unit 110 positioned on the other end in the longitudinal direction (direction of arrow A in the figure) of the device body. The positive electrode 122 is formed by a conductive member, and is electrically connected to the processing circuit 150 (see Fig. 9) arranged in the body unit 100 through a connection cable 170 (see Fig. 8) to be hereinafter described. The positive electrode 122 is arranged at substantially the middle of the left side face 116 of the detachable unit 110, and is configured such that the surface thereof is positioned slightly projecting from the outer surface of the detachable unit 110.

An outer appearance structure in the state where the detachable unit is separated from the body unit will now be described. As shown in Fig. 8, the detachable unit 110 is configured so as to be separable from the body unit 100, where the detachable unit 110 is detached from the accommodating section 108 formed in the body unit 100 in the separated state.

Engagement projections 107a, 107b are arranged on the accommodating section 108 of the body unit 100. The engagement projections 107a, 107b are respectively connected to the release buttons 107A, 107B described above by way of a link mechanism (not shown). The engagement projections 107a, 107b respectively engage engagement concave parts 113a, 114a formed in the upper face 113 and the lower face 114 of the detachable unit 110 when the detachable unit 110 is united to the body unit 100. That is, the engagement projections 107a, 107b and the engagement concave parts 113a, 114a serve as an engagement mechanism for maintaining the united state of the body unit 100 and the detachable unit 110 in a state where the detachable unit 110 is accommodated in the accommodating section 108 of the body unit 100.

The engagement projections 107a, 107b are configured to move in the direction of an arrow B in the figure by operating the release buttons 107A, 107B. Therefore, in separating the detachable unit 110 from the body unit 100, the examinee operates the release buttons 107A, 107B so that the engagement projections 107a, 107b are accommodated inside the body unit 100 in cooperation with the operation of the release buttons 107A, 107B, thereby disengaging the engagement with respect to the engagement concave parts 114a, 115a of the engagement projections 107a 107b, and enabling the detachable unit 110 to be detached from the body unit 100.

The engagement projections 107a, 107b are preferably always biased in a direction of projecting from the outer surface of the body unit 100 by a biasing member such as a bias spring. The detachment of the detachable unit 110 with respect to the body unit 100 can be easily carried out according to such a configuration.

As shown in Fig. 8, the body unit 100 and the detachable unit 110 are wire connected by a connection cable 170. The connection cable 170 is a connection member for electrically connecting the processing circuit (see Fig. 9) arranged in the body unit 100 and the positive electrode 122 arranged in the detachable unit 110, where one end is fixed to a reel 180 serving as a winding mechanism arranged in the body unit 100 and the other end is fixed to the detachable unit 110. The reel 180 is installed to be rotatable in a direction of an arrow C in the figure inside the body unit 100. Therefore, the connection cable 170 is pulled out from the body unit 100 or pulled into the body casing 100 when the reel 180 rotates.

The reel 180 is preferably always biased in a winding direction of the connection cable 170 by the biasing member of the bias spring. According to such a configuration, the connection cable 170 is smoothly winded when attaching the detachable unit 110 to the body unit 100. A wiring excelling in flexibility is preferably used for the connection cable 170 from the standpoint of winding and from the standpoint of handleability.

Fig. 9 is a functional block diagram of the portable electrocardiograph according to the present embodiment. The functional block of the portable electrocardiograph according to the present embodiment will now be described with reference to the figure.

As shown in Fig. 9, the portable electrocardiograph 1A according to the present embodiment mainly includes an electrode unit 120, an operation unit 140, and a processing circuit 150. The electrode unit 120 is configured by the negative electrode 121, the positive electrode 122, and the indifferent electrode 124. The operation unit 140 is configured by the power button 141, the measurement button 142, the menu button 143, the determination button 144, the left scroll button 145, and the right scroll button 146.

The processing circuit 150 incorporates a circuit for processing a living body electric signal detected by the electrode unit 120 to measure the same as an electrocardiographic waveform, and includes an amplifier circuit 151 for amplifying the living body electric signal detected by the electrode unit 120, a filter circuit 152 for removing noise component from the living body electric signal detected by the electrode unit 120, an A/D (Analog/Digital) converter 153 for converting an analog signal to a digital signal, a CPU (Central Processing Unit) 154 for performing various calculations, a memory 155 including a ROM (Read Only Memory) and a RAM (Random Access Memory) for storing electrocardiographic information, and a timer 156 for outputting time data timed through a timing operation to the CPU 154. The amplifier circuit 151 differentially amplifies an output voltage signal (living body electric signal) of the negative electrode 121 and the positive electrode 122 based on the output voltage signal of the indifferent electrode 124, and outputs the same. The filter circuit 152 uses a band pass filter having a pass band of between 0.5 Hz and 35 Hz and the like.

The processing circuit 150 is connected with the electrode unit 120 and the operation unit 140, and in addition, connected with the display unit 148 and the power 160. When an external storage medium is inserted to the slot for inserting the external storage medium, the external storage medium 132 is also connected to the processing circuit 150.

The CPU 154 executes an analysis process of the digital signal input from the A/D converter 153, receives a command signal from various operation buttons included in the operation unit 140, and executes a process corresponding to the received command signal. The CPU 154 executes write and readout of information to and from the memory 155, and performs display control on the display unit 148.

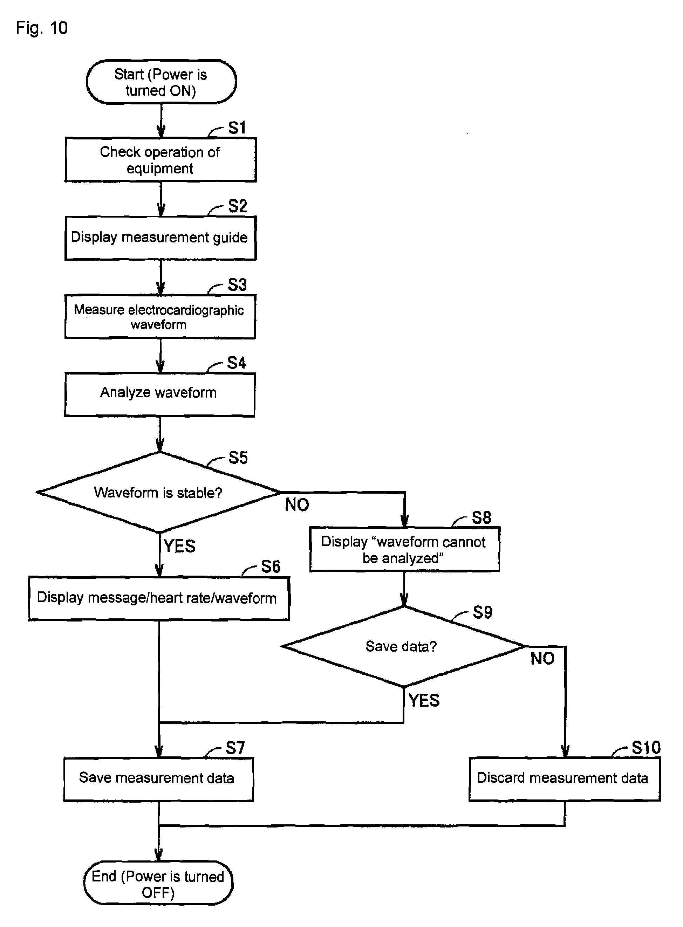

Fig. 10 is a flowchart showing a flow of processes executed in the portable electrocardiograph according to the present embodiment. The flow of processes in the portable electrocardiograph according to the present embodiment will now be described below with reference to the drawings.

The processes shown in the flowchart of Fig. 10 are stored in advance in the ROM of the memory 155 as a program, and the execution of the processes is carried out by causing the CPU 154 to read out and execute the program.

As shown in Fig. 10, when the power button 141 is pushed by the examinee and the power of the portable electrocardiograph 1A is turned ON, the operation of the equipment is checked (step S1), and the measurement guide is displayed on the display unit 148 (step S2). The display of the measurement guide includes a message etc. displaying the measuring position the examinee needs to take for measurement as guidance. The measuring position will be hereinafter described.

When the examinee pushes the measurement button 142 thereafter while taking a predetermined measuring position, measurement and analysis of the electrocardiographic waveform are executed (steps S3 and S4). The electrocardiographic waveform converted to a digital signal by the A/D converter 153 is temporarily recorded in the RAM of the memory 155. The analysis of the electrocardiographic waveform is a process of detecting presence of shape feature showing arrhythmia, ischemia etc. of cardiac muscle, presence of periodic feature showing slow pulse and fast pulse, presence of an unanalyzable waveform due to noise and baseline fluctuation, and the like from the electrocardiographic waveform converted to a digital signal, and analyzing the detection result. The measurement and the analysis of the electrocardiographic waveform may be carried out through known procedures.

Determination is made on whether or not obtained the electrocardiographic waveform is stable as a result of waveform analysis (step S5), where if determined that the waveform is stable (YES in step S5), the process proceeds to step S6, and a message etc. based on the measurement waveform, hear rate, and analysis result of the waveform is displayed on the display unit 148. In step S6, the CPU 154 edits the analysis result of the electrocardiographic waveform to a message, and displays the same on the display unit 148. In this case, the hear rate per unit time is displayed with the message. The heart rate can be acquired through a known procedure based on the electrocardiographic waveform. The process proceeds to step S7 after the process of step S6 is terminated. If determined that the electrocardiographic waveform is not stable (NO in step S6), the process proceeds to step S8, and notification that waveform analysis was not possible is displayed, and a message on whether or not to save the measurement data is displayed (step S9).

In step S9, determination is made on whether or not the examinee has selected to save the measurement data according to the message that waveform analysis was not possible, and the process proceeds to step S7 if selection is made to save the measurement data (YES in step S9). If selection is made to discard the measurement data (NO in step S9), the process proceeds to step S10.

In step S7, the CPU 154 performs a process of saving the measurement data. That is, the CPU 154 stores the current date and time data input from the timer 156, the electrocardiographic waveform data temporarily stored in the RAM, and the analysis result in correspondence to each other in a predetermined storage region of the memory 155. In step S10, the CPU 154 discards the measurement data.

The measurement and storage of the electrocardiographic waveform by the portable electrocardiograph 1A are realized by executing the series of processes in the portable electrocardiograph 1A. The portable electrocardiograph 1A according to the present embodiment has a function of reading out the measurement result and the analysis result of the electrocardiographic waveform saved in step S7 and displaying the same on the display unit 148.

Fig. 11 and Fig. 12 are views showing display examples of the measurement result in the display unit of the portable electrocardiograph according to the present embodiment. The display example of the portable electrocardiograph according to the present embodiment will be described below with reference to the drawings.

As shown in Fig. 11, a reduced waveform 162 of the entire waveform over the entire measurement period is displayed at the lower level of the screen at the display starting point, and an enlarged waveform 164 of for example, two seconds after the start of measurement is displayed at the upper level thereof in the display unit 148. A scale bar 165 showing the length of the measurement period is displayed below the reduced waveform 162 close to the reduced waveform 162. A pointer 166 for instructing which waveform of which part of the measurement period the enlarged waveform 164 is related to is displayed on the scale bar 165. Change on which waveform of which part of the entire measurement period to display as the enlarged waveform 164 can be made by operating the right scroll button 146 and the left scroll button 145, and moving the pointer 166 on the scale bar 165. The reduced waveform 164 thus can be displayed in units of arbitrary two seconds of the entire measurement period.

When the examinee pushes the determination button 144 after checking the electrocardiographic waveform on the screen of Fig. 11, the CPU 154 reads out the data of the analysis result corresponding to the electrocardiographic waveform being displayed from the memory 155, edits the same to a message, and displays the edited message on the display unit 148 as shown in Fig. 12.

In the portable electrocardiograph 1A according to the present embodiment described above, the device body is divided into the body unit 100 and the detachable unit 110, and the detachable unit 110 is detachably attached to the body unit 100. Therefore, two states including a state where the detachable unit 110 is attached to the body unit 100 and a state where the detachable unit 110 is detached from the body unit 100 can be taken. The electrocardiographic waveform can be measured in either of the above two states in the portable electrocardiograph 1A according to the present embodiment.

Specifically, in the state where the detachable unit 110 is united to the body unit 110 as shown in Fig. 1 to Fig. 7 and in the state where the detachable unit 110 is separated from the body unit 100 as shown in Fig. 8, the negative electrode 121 and the indifferent electrode 124 are exposed at the outer surface (exposed right side face 105 of the body unit 100) of the device body, and the positive electrode 122 is exposed at the outer surface (exposed left side face 116 of the detachable unit 110) of the device body. The electrocardiographic waveform thus can be measured by measuring a potential difference generated between the negative electrode 121 and the positive electrode 122 both in the state where the detachable unit 110 is united to the body unit 100 and in the state where the detachable unit 110 is separated from the body unit 100 by bringing the negative electrode 121, the positive electrode 122, and the indifferent electrode 124 into contact with the body surface.

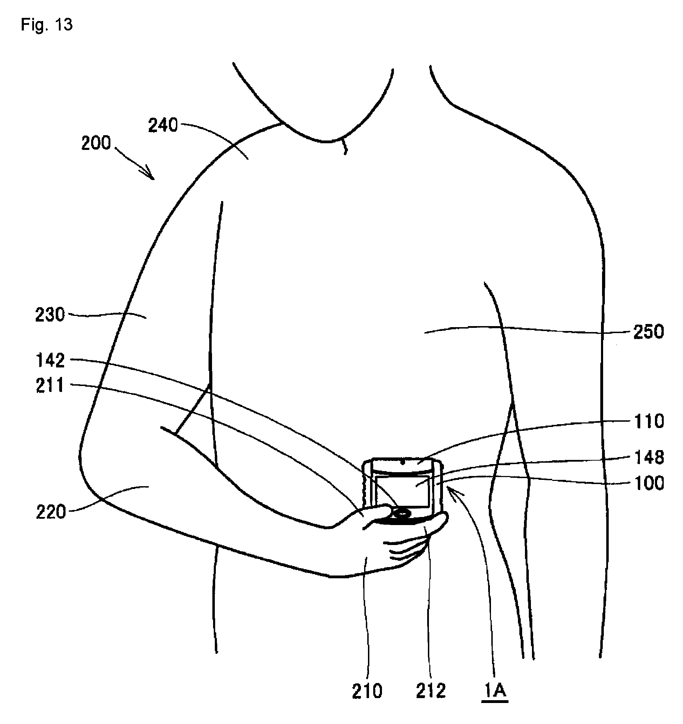

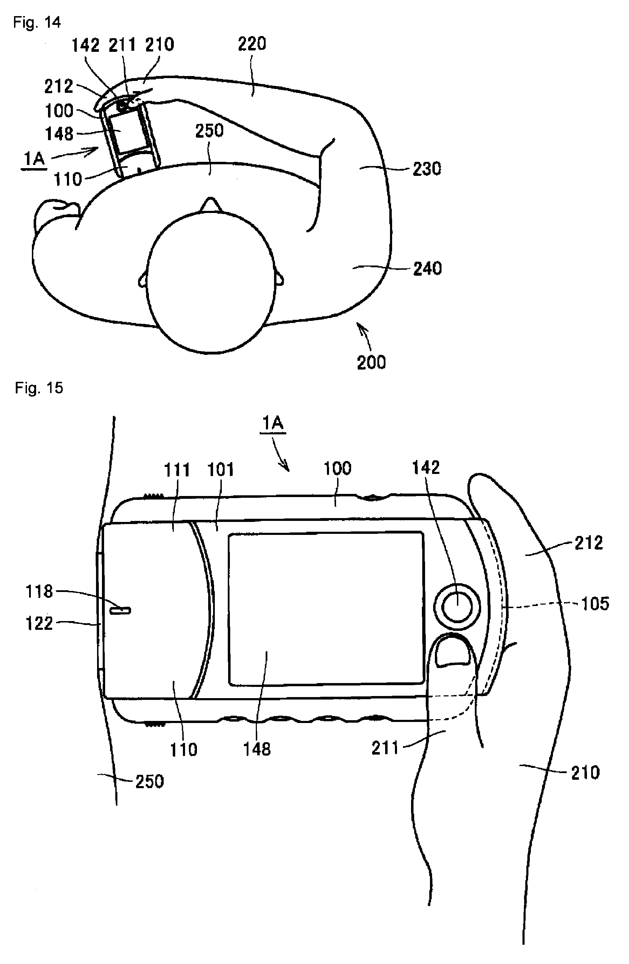

Fig. 13 and Fig. 14 are views showing a measuring position to be taken by the examinee when measuring the electrocardiographic waveform with the detachable unit united to the body unit, and Fig. 15 is a view showing a gripping state by the right hand of the examinee when the examinee takes the measuring position. The measuring position to be taken by the examinee and the measuring operation performed when measuring the electrocardiographic waveform with the detachable unit united to the body unit in the portable electrocardiograph of the present embodiment will be described below with reference to the drawings.

As shown in Fig. 13 and Fig. 14, the examinee 200 directly brings the positive electrode 122 arranged at the other end (left side face 116 of the detachable unit 110) of the device body into contact with the skin at the fifth intercostal space in the anterior axillary line positioned at the lower part on the left side of the chest region 250 with the alignment mark 118 formed in the detachable unit 110 as a mark while gripping, with the right hand 210, the portion closer to one end of the device body (portion closer to right side face 105 of the body unit 100) formed by uniting the detachable unit 110 to the body unit 100. The measurement button 142 arranged on the front face 101 of the body unit 100 is then pushed with the thumb 211 of the right hand 210. After the measurement button 142 is pushed, a state where the portion closer to one end of the device body is gripped with the right hand 210 and the other end of the device body is pressed against the relevant position of the chest region 250 is maintained until the measurement of the electrocardiographic waveform is terminated.

In this case, as shown in Fig. 15, the examinee 200 grips the body unit 100 with the right hand 210 so that the front face of the device body of the portable electrocardiograph 1A is faced upward, the forefinger 212 of the right hand 210 is placed on the right side face 105 of the body unit 100 or the right side face of the device body, the thumb 211 of the right hand 210 is pressed against the front face 101 of the body unit 100, and the middle finger of the right hand 210 is pressed against the rear face 102 of the body unit 100, whereby the body unit 100 is gripped so as to be sandwiched in the up and down direction. Here, the forefinger 212 of the right hand 210 is lightly bent so as to lie along the curved right side face 105 of the body unit 100 and inserted into the concave part 105a formed in the right side face 105, so as to be brought into contact with the negative electrode 121 and the indifferent electrode 124 arranged in the concave part 105a.

The negative electrode 121 and the indifferent electrode 124 positioned on the right side face 105 of the body unit 100 of the portable electrocardiograph 1A are brought into contact with the forefinger 212 of the right hand 210 of the examinee 200, and the positive electrode 122 positioned on the left side face 116 of the detachable unit 110 is brought into contact with the chest region 250 of the examinee 200 by taking the measuring position described above. A circuit for measuring the electrocardiographic waveform is configured in the body of the examinee 200 in the order of the right hand 210 brought into contact with the negative electrode 121 and the indifferent electrode 124, the forearm 220 not in contact with the chest region 250, the upper arm 230 similarly not in contact with the chest region 250, the right shoulder 240, and the chest region 250 pressed by the positive electrode 122. Therefore, the electrocardiographic waveform can be measured by maintaining the measuring position. A case of performing the measurement in an uprising position is shown in Fig. 13 and Fig. 14, but measurement may be performed in the lying position or a seating position.

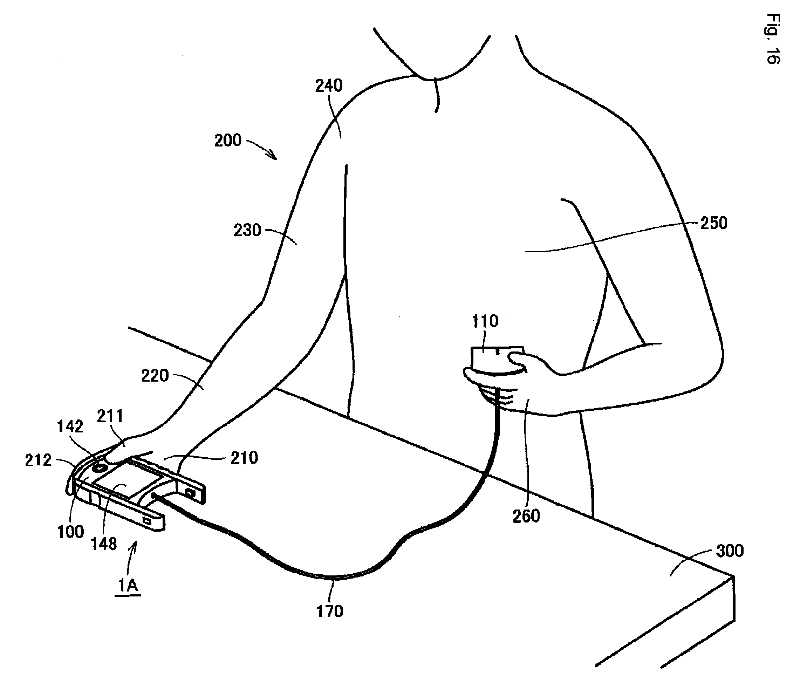

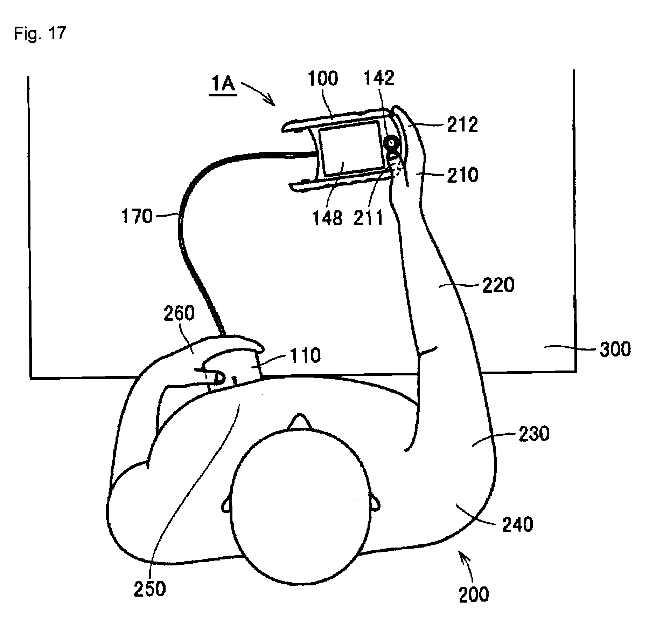

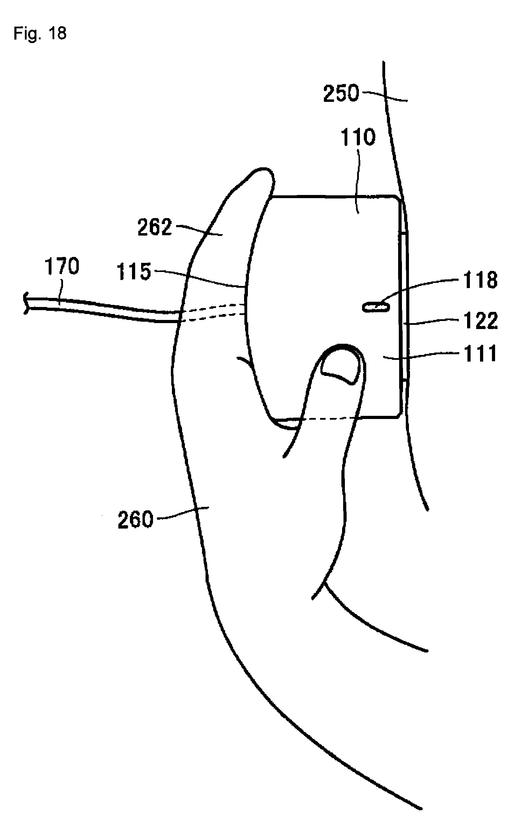

Fig. 16 and Fig. 17 are views showing a measuring position to be taken by the examinee when measuring the electrocardiographic waveform with the detachable unit separated from the body unit, and Fig. 18 is a view showing a gripping state of the detachable unit by the left hand of the examinee when the examinee takes the measuring position. The measuring position to be taken by the examinee and the measuring operation performed when measuring the electrocardiographic waveform with the detachable unit separated from the body unit in the portable electrocardiograph according to the present embodiment will be described below with reference to the drawings.

As shown in Fig. 16 and Fig. 17, the examinee 200 grips the portion closer to the right side face 105 of the body unit 100 with the right hand 210, grips the portion closer to the right side face 115 of the detachable unit 110 separated from the body unit 100 with the left hand 260, and directly brings the positive electrode 122 arranged on the left side face 116 of the detachable unit 110 into contact with the skin at the fifth intercostal space in the anterior axillary line positioned at the lower part on the left side of the chest region 250 with the alignment mark 118 as the mark. The measurement button 142 arranged on the front face 101 of the body unit 100 is then pushed with the thumb 211 of the right hand 210. After the measurement button 142 is pushed, a state where the portion closer to the right side face 105 of the body unit 100 is gripped with the right hand 210, the portion closer to the right side face 115 of the detachable unit 110 is gripped with the left hand 260, and the left side face 116 of the detachable unit 110 is pressed against the relevant position of the chest region 250 is maintained until the measurement of the electrocardiographic waveform is terminated.

In this case, the examinee 200 grips the portion closer to the right side face 105 of the body unit 100 in a state same as the state shown in Fig. 15, so that the forefinger 212 of the right hand 210 is brought into contact with the negative electrode 121 and the indifferent electrode 124, the portion closer to the right side face 115 of the detachable unit 110 is gripped with the left hand 260, and the left side face 116 of the detachable unit 110 is pressed against the relevant position of the chest region 250, shown in Fig. 18.

The negative electrode 121 and the indifferent electrode 124 positioned on the right side face 105 of the body unit 100 of the portable electrocardiograph 1A are brought into contact with the forefinger 212 of the right hand 210 of the examinee 200, and the positive electrode 122 positioned on the left side face 116 of the detachable unit 110 is in contact with the chest region 250 of the examinee 200 by taking the measuring position described above. A circuit for measuring the electrocardiographic waveform is configured in the body of the examinee 200 in the order of the right hand 210 in contact with the negative electrode 121 and the indifferent electrode 124, the forearm 220 not in contact with the chest region 250, the upper arm 230 similarly not in contact with the chest region 250, the right shoulder 240, and the chest region 250 pressed by the positive electrode 122. Therefore, the electrocardiographic waveform can be measured by maintaining the measuring position.

When taking this measuring position, the forearm 220 of the right hand 210 gripping the body unit 100 is preferably placed on a table such as a desk 300, as shown in Fig. 16 and Fig. 17. Since the myoelectric generated in the right hand gripping the body unit 100 will no longer superimpose the measurement data by placing the forearm 220 of the right hand 210 on the table, the electrocardiographic waveform can be measured at high precision. Furthermore, if the examinee 200 sits on a chair etc. in this case, a comfortable position without load being applied on the body can be realized. The measurement may also be carried out in a lying position.

When this measuring position is taken, the body unit 100 can be disposed at a position spaced apart towards the front from the chest region 250, and thus the visibility of the display unit 148 can be enhanced compared to when the measuring position shown in Fig. 13 and Fig. 14 is taken. Therefore, whether or not the measurement is normally carried out can be checked on the display unit 148 during the measurement, and failure in data acquisition due to operation mistake can be prevented more easily.

The electrocardiographic waveform can be obtained by measuring the potential difference generated between the negative electrode 121 and the positive electrode 122 in the two states including a state where the detachable unit 110 is united to the body unit 100 and a state where the detachable unit 110 is separated from the body unit 100 according to the portable electrocardiograph 1A of the present embodiment described above. Since the examinee himself/herself can select one measuring position from the two measuring positions according to the situation, the electrocardiographic waveform can be rapidly and easily measured when a certain symptom to be measured occurs. In the state where the detachable unit 110 is separated from the body unit 100, the electrocardiographic waveform can be measured by having the caretaker, the doctor, and the like subsidiarily support the detachable unit 110 when a symptom such as palpitation and short breath is severe and it is difficult for the examinee himself/herself to measure. Therefore, a portable electrocardiograph extremely excelling in handleability can be achieved.

### (Second embodiment)

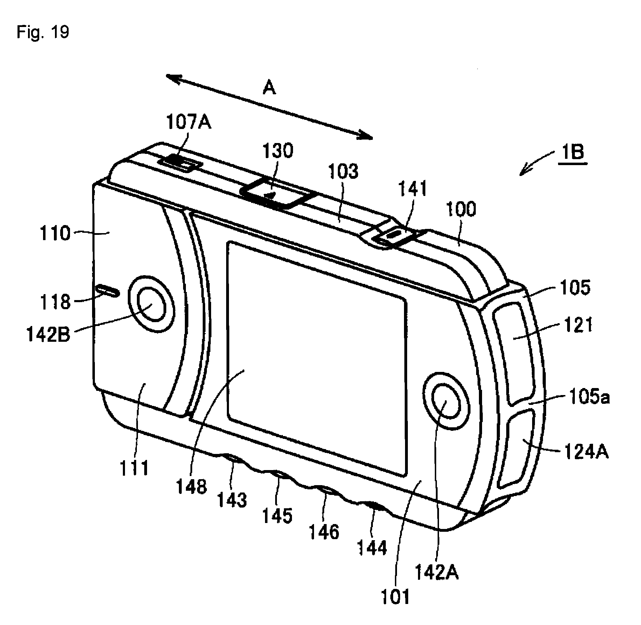

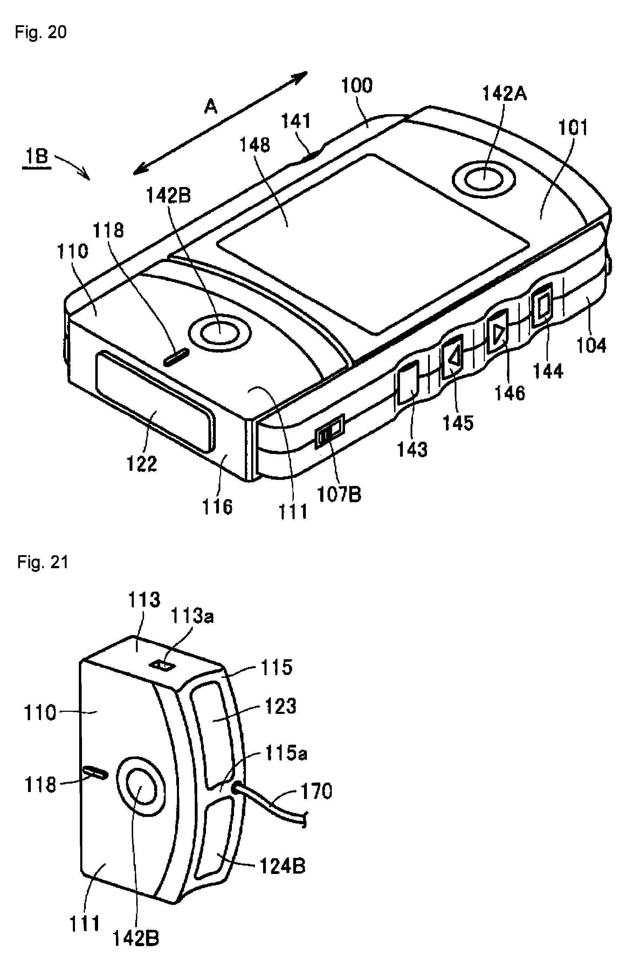

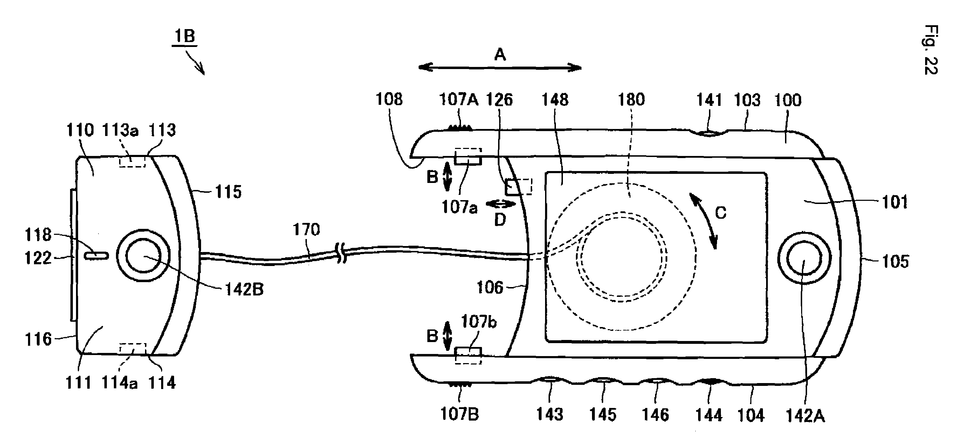

Fig. 19 and Fig. 20 are perspective views showing the state where the body unit and the detachable unit are united to each other in a portable electrocardiograph according to a second embodiment of the present invention. Fig. 21 is a perspective view of the detachable unit in the state where the body unit is separated from the detachable unit in the portable electrocardiograph according to the present embodiment. Fig. 22 is a front view showing the state where the detachable unit is detached from the body unit in the portable electrocardiograph according to the present embodiment. In the figure, same reference numerals are denoted for the portions similar to the portable electrocardiograph according to the first embodiment described above, and the description thereof will not be repeated herein.

First, an outer appearance structure of the state where the detachable unit is united to the body unit in the portable electrocardiograph according to the present embodiment will be described. As shown in Fig. 19 and Fig. 20, a portable electrocardiograph 1 B according to the present embodiment includes the body unit 100 serving as the first unit and the detachable unit 110 serving as the second unit, similar to the portable electrocardiograph 1A according to the first embodiment described. The detachable unit 110 is detachably attached to the body unit 100.

The body unit 100 of the portable electrocardiograph 1 B according to the present embodiment substantially has a configuration similar to the body unit 100 of the portable electrocardiograph 1A according to the first embodiment, where the display unit 148 and the first measurement button 142A are arranged on the front face 101, and the first negative electrode 121 serving as the first electrode and a first indifferent electrode 124A are arranged on the right side face 105. Various operation buttons 141, 143 to 146, the open/close cover 130, and the release buttons 107A, 107B are arranged on the upper face 103 and the lower face 104 of the body unit 100. The accommodating section 108 for receiving the detachable unit 110 is arranged on the left side face 106 side of the body unit 100 (see Fig. 22).

As shown in Fig. 19 to Fig. 21, the detachable unit 110 of the portable electrocardiograph 1 B according to the present embodiment has substantially the same configuration as the detachable unit 110 of the portable electrocardiograph 1A according to the first embodiment described above, but the configuration differs in that a second negative electrode 123 serving as a third electrode and a second indifferent electrode 124B are arranged on the right side face 115. As shown in Fig. 21, the right side face 115 of the detachable unit 110 has a smoothly curved shape so that the forefinger of the right hand of the examinee fits thereto when the examinee takes the measuring position to be hereinafter described.

The second electrode 123 and the second indifferent electrode 124B are respectively formed by a conductive member, and are electrically connected to the processing circuit 150 arranged in the body unit 100 by way of the connection cable 170. The second negative electrode 123 and second indifferent electrode 124B are disposed so that the surfaces thereof are exposed at the outer surface of the detachable unit 110 in the concave part 115a formed in the right side face 115 of the detachable unit 110. The second negative electrode 123 is positioned closer to the upper face 113 of the right side face 115, and the second indifferent electrode 123B is positioned closer to the lower face 114 of the right side face 115.

The left side face 106 of the body unit 100 and the right side face 115 of the detachable unit 110 face each other in the state where the detachable unit 110 is united to the body unit 100. In this state, the second negative electrode 123 and the second indifferent electrode 124B arranged on the right side face 115 of the detachable unit 110 are both covered by the left side face 106 of the body unit 100.

A second measurement button 142B is arranged on the front face 111 of the detachable unit 110. The second measurement button 142B is an operation button for starting the measurement of the electrocardiographic waveform, similar to the first measurement button 142A described above.

In the portable electrocardiograph according to the present embodiment, an outer appearance structure of the state where the detachable unit is separated from the body unit will be described. As shown in Fig. 22, the detachable unit 110 is configured to be separable from the body unit 100, where the detachable unit 110 is detached from the accommodating section 108 arranged in the body unit 100 in the separated state. Similar to the portable electrocardiograph 1A according to the first embodiment described above, the united state of the units 100, 110 is maintained by the engagement projections 107a, 107b and the engagement concave parts 113a, 114a serving as the engagement mechanism in the portable electrocardiograph 100B according to the present embodiment, where the release buttons 107A, 107B connected to the engagement projections 107a, 107b by way of a link mechanism (not shown) need to be operated to disengage the engagement of the engagement mechanism.

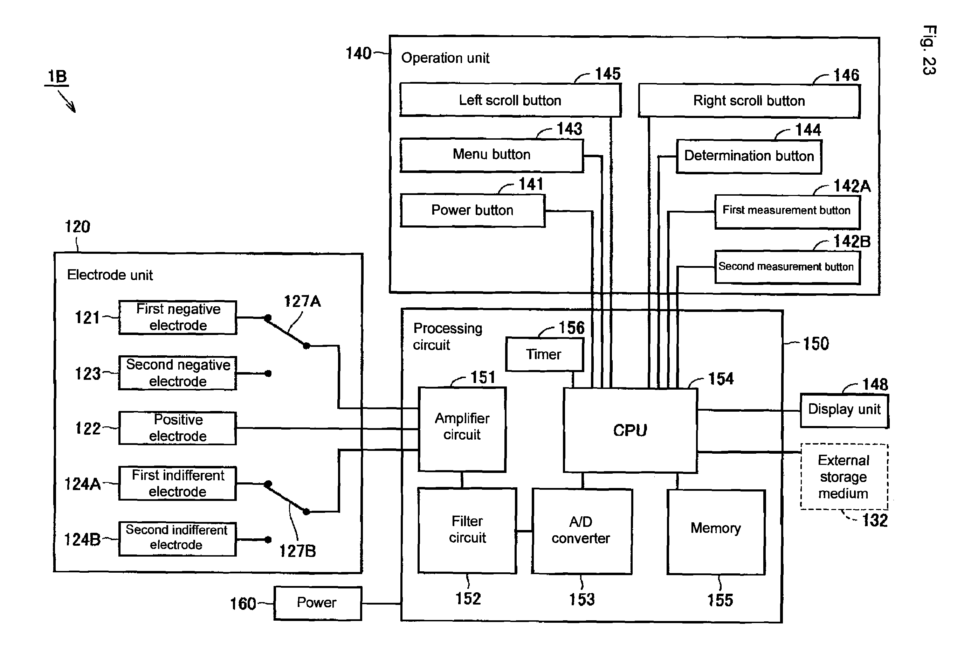

In the portable electrocardiograph 1 B according to the present embodiment, a detection button 126 serving as a detection unit for detecting whether or not the detachable unit 110 is accommodated in the accommodating section 108 of the body unit 100 is arranged at a predetermined position in the accommodating section 108 of the body unit 100. The detection button 126 is arranged to project from the wall face of the accommodating section 108 when not accommodated. The detection button 126 is configured to be movable in a direction indicated by an arrow D in the figure, but is always biased in the projecting direction by a bias member (not shown) of the bias spring. When the detachable unit 110 is accommodated in the accommodating section 108, accommodation of the detachable unit 110 in the accommodating section 108 of the body unit 100 is detected when the detection button 126 is pushed and pushed into the body unit 100. The detection button 126 cooperatively operates with relays 127A, 127B (see Fig. 23) serving as a switch unit to be hereinafter described.

In the state where the detachable unit 110 is separated from the body unit 100, the right side face 115 of the detachable unit 110 is exposed. In this state, the second negative electrode 123 and the second indifferent electrode 124B arranged on the right side face 115 of the detachable unit 110 are thus both exposed.

Fig. 23 is a functional block diagram of the portable electrocardiograph according to the present embodiment. The functional block of the portable electrocardiograph according to the present embodiment will now be described with reference to the drawings. As shown in Fig. 23, the portable electrocardiograph 1 B according to the present embodiment mainly includes the electrode unit 120, the operation unit 140, and the processing circuit 150. The configuration of the processing circuit 150 is similar to the portable electrocardiograph 1A in the first embodiment described above.

The electrode unit 120 is configured by the first negative electrode 121, the second negative electrode 123, the positive electrode 122, the first indifferent electrode 124A, and the second indifferent electrode 124B. The first negative electrode 121 and the second negative electrode 123 are alternatively connected to the amplifier circuit 151 by the relay 127A. The first indifferent electrode 124A and the second indifferent electrode 124B are alternatively connected to the amplifier circuit 151 by the relay 127B. The relays 127A, 127B cooperatively operate with the detection button 126 as described above, and are switched to connect the first negative electrode 121 and the first indifferent electrode 124A to the amplifier circuit 151 in the state where the detachable unit 110 is united to the body unit 100, and to connect the second negative electrode 123 and the second indifferent electrode 124B to the amplifier circuit 151 in the state where the detachable unit 110 is separated from the body unit 100.

The operation unit 140 is configured by the power button 141, the first measurement button 142A, the second measurement button 142B, the menu button 143, the determination button 144, the left scroll button 145, and the right scroll button 146. The first measurement button 142A and the second measurement button 142B are both operation buttons for starting the measurement of the electrocardiographic waveform, where when either one of the buttons is pushed, a command to start the measurement is output to the CPU 154.

In the portable electrocardiograph 1 B of the present embodiment described above, the device body is divided to the body unit 100 and the detachable unit 110, and the detachable unit 110 is detachably attached to the body unit 100. Therefore, two states including the state where the detachable unit 110 is attached to the body unit 100 and the state where the detachable unit 110 is detached from the body unit 100 can be taken. In the portable electrocardiograph 1 B according to the present embodiment, the electrocardiographic waveform can be measured in either of the two states descried above.

Specifically, the first negative electrode 121 and the indifferent electrode 124A are exposed at the outer surface of the device body (exposed right side face 105 of the body unit 100) in the state where the detachable unit 110 is united to the body unit 100 as shown in Fig. 19 and Fig. 20, and the positive electrode 122 is exposed at the outer surface of the device body (exposed left side face 116 of the detachable unit 110). In the state where the detachable unit 110 is separated from the body unit 110 as shown in Fig. 22, the second negative electrode 123 and the second indifferent electrode 124B are exposed at the outer surface of the device body (right side face 115 of the detachable unit 110 exposed by separating the detachable unit 110 from the body unit 110) in addition thereto. The electrocardiographic waveform thus can be measured by measuring the potential difference generated between the first negative electrode 121 and the positive electrode 122 or the potential difference generated between the second negative electrode 123 and the positive electrode 122 both in the state where the detachable unit 110 is united to the body unit and in the state where the detachable unit 110 is separated from the body unit 100 by bringing the first negative electrode 121, the positive electrode 122 and the first indifferent electrode 124A or the second negative electrode 123, the positive electrode 122, and the second indifferent electrode 124B into contact with the body surface.

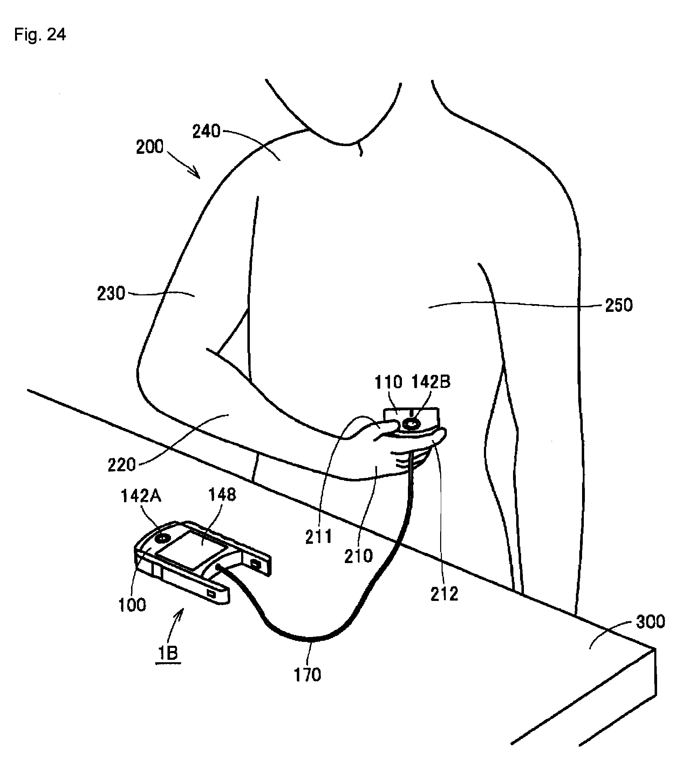

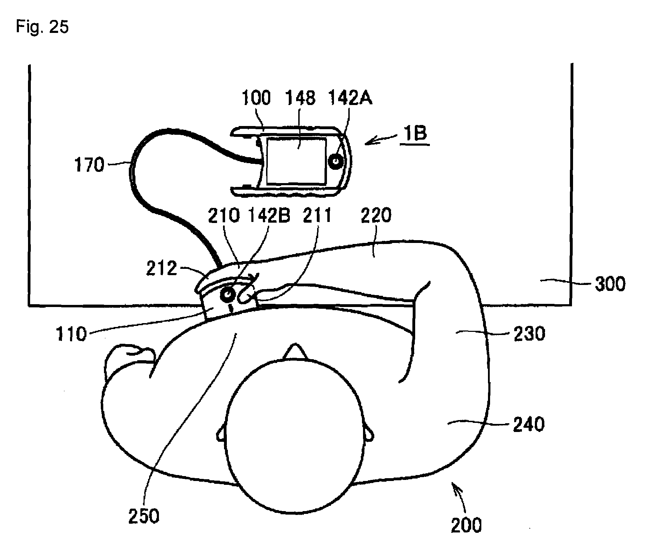

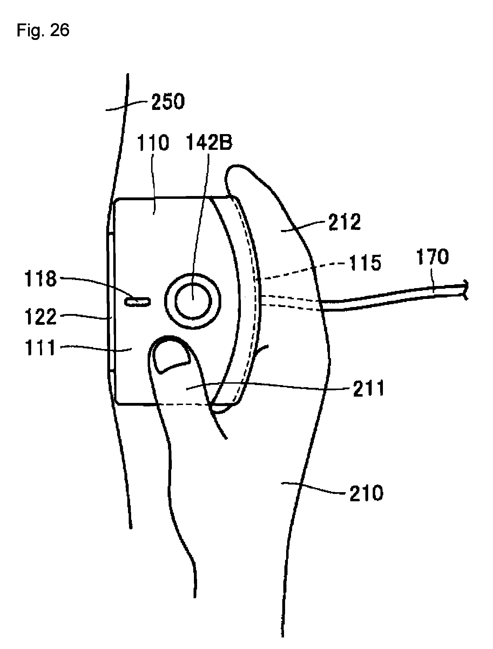

The measuring position to be taken by the examinee and the measuring operation performed in the portable electrocardiograph according to the present embodiment will now be described. Fig. 24 and Fig. 25 are perspective view and top view, respectively, showing a measuring position to be taken by the examinee when measuring the electrocardiographic waveform with the detachable unit separated from the body unit in the portable electrocardiograph according to the present embodiment, and Fig. 26 is a view showing a gripped state of the detachable unit by the right hand of the examinee when the examinee takes the measuring position.

When measuring the electrocardiographic waveform with the detachable unit 100 united to the body unit 100, the examinee takes a position similar to the measuring position described using Fig. 13 to Fig. 15 in the first embodiment described above. That is, the examinee directly brings the positive electrode 122 arranged at the other end (left side face 116 of the detachable unit 110) of the device body into contact with the skin at the fifth intercostal space in the anterior axillary line positioned at the lower part of the left side of the chest region 250 with the alignment mark 118 formed in the detachable unit 110 as a mark while gripping, with the right hand 210, the portion closer to one end of the device body (portion closer to right side face 105 of the body unit 100) formed by uniting the detachable unit 110 to the body unit 100. The measurement button 142A arranged on the front face 101 of the body unit 100 is then pushed with the thumb 211 of the right hand 210. After the measurement button 142A is pushed, a state where the portion closer to one end of the device body is gripped with the right hand 210 and the other end of the device body is pressed against the relevant position of the chest region 250 is maintained until the measurement of the electrocardiographic waveform is terminated.

In this case, the examinee 200 grips the body unit 100 with the right hand 210 so that the front face of the device body of the portable electrocardiograph 1 B is faced upward, the forefinger 212 of the right hand 210 is placed on the right side face 105 of the body unit 100 or the right side face of the device body, the thumb 211 of the right hand 210 is pressed against the front face 101 of the body unit 100, and the middle finger of the right hand 210 is pressed against the rear face 102 of the body unit 100, where the body unit 100 is gripped so as to be sandwiched in the up and down direction. Here, the forefinger 212 of the right hand 210 is lightly bent so as to lie along the curved right side face 105 of the body unit 100 and inserted into the concave part 105a formed in the right side face 105, so as to be brought into contact with the first negative electrode 121 and the first indifferent electrode 124A arranged in the concave part 105a.

The first negative electrode 121 and the first indifferent electrode 124A positioned on the right side face 105 of the body unit 100 of the portable electrocardiograph 1 B are brought into contact with the forefinger 212 of the right hand 210 of the examinee 200, and the positive electrode 122 positioned on the left side face 116 of the detachable unit 110 is brought into contact with the chest region 250 of the examinee 200 by taking the measuring position described above. A circuit for measuring the electrocardiographic waveform is configured in the body of the examinee 200 in the order of the right hand 210 in contact with the first negative electrode 121 and the first indifferent electrode 124A, the forearm 220 not in contact with the chest region 250, the upper arm 230 similarly not in contact with the chest region 250, the right shoulder 240, and the chest region 250 pressed by the positive electrode 122.

When measuring the electrocardiographic waveform with the detachable unit 110 separated from the body unit 100, the examinee 200 places the body unit 100 on the table such as the desk 300, and directly brings the positive electrode 122 arranged on the left side face 116 of the detachable unit 110 into contact with the skin at the fifth intercostal space in the anterior axillary line positioned at the lower part of the left side of the chest region 250 with the alignment mark 118 as a mark while gripping, with the right hand 210, the portion closer to right side face 115 of the detachable unit 110, as shown in Fig. 24 and Fig. 25. The measurement button 142B arranged on the front face 111 of the detachable unit 100 is then pushed with the thumb 211 of the right hand 210. After the measurement button 142B is pushed, a state where the portion closer to the right side face 115 of the detachable unit 110 is gripped with the right hand 210, and the left side face 116 of the detachable unit 110 is pressed against the relevant position of the chest region 250 is maintained until the measurement of the electrocardiographic waveform is terminated.

In this case, as shown in Fig. 26, the examinee 200 grips the portion closer to the right side face 115 of the detachable unit 110 with the right hand 210, places the forefinger 212 of the right hand 210 on the right side face 115, presses the thumb 211 of the right hand 210 against the front face 111 of the detachable unit 110 and presses the middle finger of the right hand 210 against the rear face 112 of the detachable unit 110, and grips the detachable unit 110 so as to be sandwiched in the up and down direction. Here, the forefinger 212 of the right hand 210 is lightly bent so as to lie along the curved right side face 115 of the detachable unit 110 and inserted into the concave part 115a formed in the right side face 115, so as to be brought into contact with the second negative electrode 123 and the second indifferent electrode 124B arranged in the concave part 115a.

The second negative electrode 123 and the second indifferent electrode 124B positioned on the right side face 115 of the detachable unit 110 of the portable electrocardiograph 1A are brought into contact with the forefinger 212 of the right hand 210 of the examinee 200, and the positive electrode 122 positioned on the left side face 116 of the detachable unit 110 is brought into contact with the chest region 250 of the examinee 200 by taking the measuring position described above. A circuit for measuring the electrocardiographic waveform is configured in the body of the examinee 200 in the order of the right hand 210 in contact with the second negative electrode 123 and the second indifferent electrode 124B, the forearm 220 not in contact with the chest region 250, the upper arm 230 similarly not in contact with the chest region 250, the right shoulder 240, and the chest region 250 pressed by the positive electrode 122. In Fig. 24 and Fig. 25, a case where the body unit 100 of the portable electrocardiograph 1 B is placed on the desk 300 is shown, but measurement may be made by gripping the body unit 100 with the left hand or measurement may be made in a lying position.

According to the portable electrocardiograph 1 B of the present embodiment described above, the electrocardiographic waveform can be obtained by measuring the potential difference generated between the first negative electrode 121 and the positive electrode 122 in the state where the detachable unit 110 is united to the body unit 100, and the electrocardiographic waveform can be obtained by measuring the potential difference generated between the second negative electrode 123 and the positive electrode 122 in the state where the detachable unit 110 is separated from the body unit 100. Since the examinee himself/herself can select one measuring position from the two measuring positions according to the situation, the electrocardiographic waveform can be rapidly and easily measured when a certain symptom to be measured occurred. Therefore, a portable electrocardiograph extremely excelling in handleability can be achieved. Furthermore, when making a measurement in the state where the detachable unit 110 is separated from the body unit 100, the detachable unit 110 to be supported by the right hand 210 is greatly miniaturized and lightened with respect to the body unit 100, and thus the load on the examinee in taking the measuring position is greatly alleviated. A stable measurement of the electrocardiographic waveform thus becomes possible.

In the portable electrocardiograph 1 B according to the present embodiment, the electrode not used for the measurement of the electrocardiographic waveform of the first negative electrode 121 and the third negative electrode 123 is separated from the processing circuit arranged in the body unit 100 by the relays 127A, 128B, in conjunction with the detection button 126 for detecting whether the body unit 100 and the detachable unit 110 are united to each other or are separated from each other. Thus, noise is prevented from superimposing the measurement data and measurement of high accuracy becomes possible, and furthermore, the switching task of the electrode to be used is not forced on the examinee. A portable electrocardiograph excelling in handleability and capable of reliably switching the electrode to be used is obtained.

In the portable electrocardiograph 1 B according to the present embodiment, the measurement button is arranged both in the body unit 100 and in the detachable unit 110. Thus, the examinee himself/herself can select and operate the measurement button that is easier to operate in the respective measuring position, and a portable electrocardiograph excelling in handleability can be obtained.

In the present embodiment, a case in which the relays 127A, 127B or the switching units operate in conjunction with the detection button 126 for detecting whether or not the body unit 100 and the detachable unit 110 are united to each other or are separated from each other has been described, but the detection button 126 may be omitted and a manually operable switching button serving as another switching unit may be arranged in the device body of the portable electrocardiograph. According to such a configuration, the electrocardiographic waveform can be measured using the first negative electrode 121 arranged in the body unit 100 without using the second negative electrode 123 arranged in the detachable unit 110 in the state where the detachable unit 110 is separated from the body unit 100. That is, according to the relevant configuration, the measuring position described using Fig. 16 to Fig. 18 in the first embodiment can be taken. In this case, the electrocardiographic waveform can be measured by having the caretaker, the doctor, or the like subsidiarily support the detachable unit 110.

### (Third embodiment)

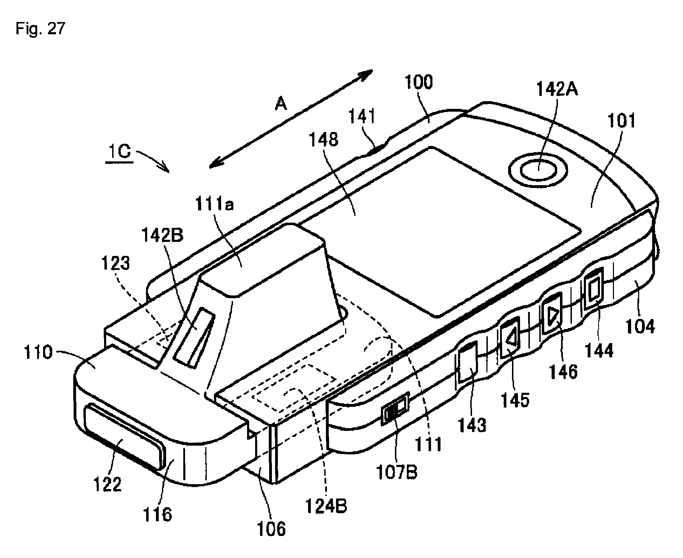

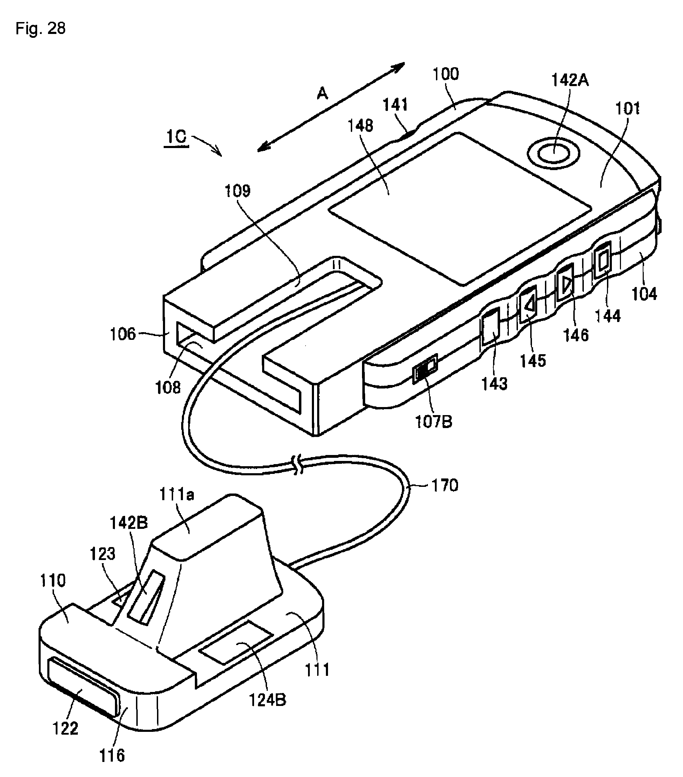

Fig. 27 and Fig. 28 are perspective views of a portable electrocardiograph according to a third embodiment of the present invention, where Fig. 27 is a view showing a state where the detachable unit is united to the body unit, and Fig. 28 is a view showing a state where the detachable unit is separated from the body unit. Same reference numerals are denoted for the portions similar to the second embodiment, and the description thereof will not be repeated herein.

As shown in Fig. 27 and Fig. 28, the portable electrocardiograph 1C according to the present embodiment includes the body unit 100 serving as the first unit and the detachable unit 110 serving as the second unit, similar to the portable electrocardiograph 1 B according to the second embodiment. The detachable unit 110 is detachably attached to the body unit 100.

The body unit 100 of the portable electrocardiograph 1C according to the present embodiment has substantially the same configuration as the body unit 100 of the portable electrocardiograph 1B of the second embodiment, where the display unit 148 and the first measurement button 142A are arranged on the front face 101, and the first negative electrode 121 serving as the first electrode and the first indifferent electrode 124A are arranged on the right side face 105. Various buttons 141, 143 to 146, the open/close cover 130, and the release buttons 107A, 107B are arranged on the upper face 103 and the lower face 104 of the body unit 100. The accommodating section 108 for receiving the detachable unit 110 is arranged on the left side face 106 side of the body unit 100, and a cutout part 109 to which a gripping part 111a of the detachable unit 110 can be inserted is formed at the front face 101 of the portion corresponding to the accommodating section 108.

The detachable unit 110 includes the gripping part 111 a projecting towards the outer side from the front face 111. The gripping part 111a is a portion to be gripped by the examinee when the examinee uses the detachable unit 110 separated from the body unit 100. The positive electrode 122 serving as the second electrode is arranged on the left side face 116 of the detachable unit 110, and a positive electrode (not shown) is arranged on the rear face (face on the side opposite to the upper face 111) of the detachable unit 110 (not shown). The second negative electrode 123 serving as the third electrode and the second indifferent electrode 124B are arranged on the front face 111 at the portion not arranged with the gripping part 111a of the detachable unit 110.
The second measurement button 142B is arranged at a predetermined position of the gripping part 111a.

As shown in Fig. 27, the detachable unit 110 is accommodated in the accommodating section 108 of the body unit 100 by inserting the gripping part 111a of the detachable unit 110 to the cutout part 109 of the body unit 100 in the state where the detachable unit 110 is united to the body unit 100. In this state, the second negative electrode 123 and the second indifferent electrode 124B arranged on the front face 111 of the detachable unit 110 are both covered by the front face 101 of the body unit 100. Therefore, when measuring the electrocardiographic waveform with the detachable unit 110 united to the body unit 100, the portion closer to the right side face 105 of the body unit 100 positioned at one end of the device body is gripped with the right hand, the forefinger of the right hand is brought into contact with the first negative electrode 121 and the first indifferent electrode 124A arranged on the right side face 105 of the body unit 100, and the positive electrode 122 arranged on the left side face 116 of the detachable unit 110 positioned at the other end of the device body is pressed against a predetermined position of the chest region to measure the electrocardiographic waveform.

As shown in Fig. 28, when the detachable unit 110 is separated from the body unit 100, the front face 111 of the detachable unit 110 is exposed, and the second negative electrode 123 and the second indifferent electrode 124B arranged on the front face 111 of the detachable unit 110 are both exposed. Therefore, the right hand is brought into contact with the second negative electrode 123 and the second indifferent electrode 124B arranged on the front face 111 of the detachable unit 110 while gripping the gripping part 111a of the detachable unit 110 with the right hand, and the positive electrode arranged on the rear face of the detachable unit 110 is pressed against a predetermined position of the chest region to measure the electrocardiographic waveform.

The present invention is applicable even when the shape of the detachable unit 110 is changed or the position formed with the second negative electrode 123 and the second indifferent electrode 124 arranged on the detachable unit 110 is changed. Therefore, the present invention can be applied even when the shape of the detachable unit 110 is changed to various shapes such as a pen type, mouse type, stethoscope type, and the like by changing the electrode forming position in various ways.

### (Fourth embodiment)

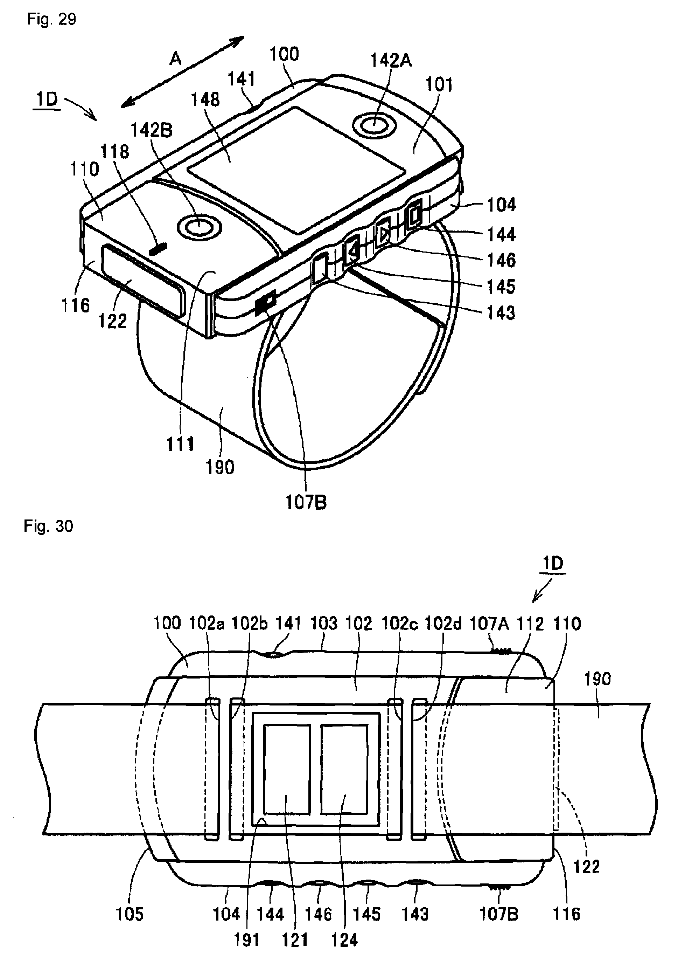

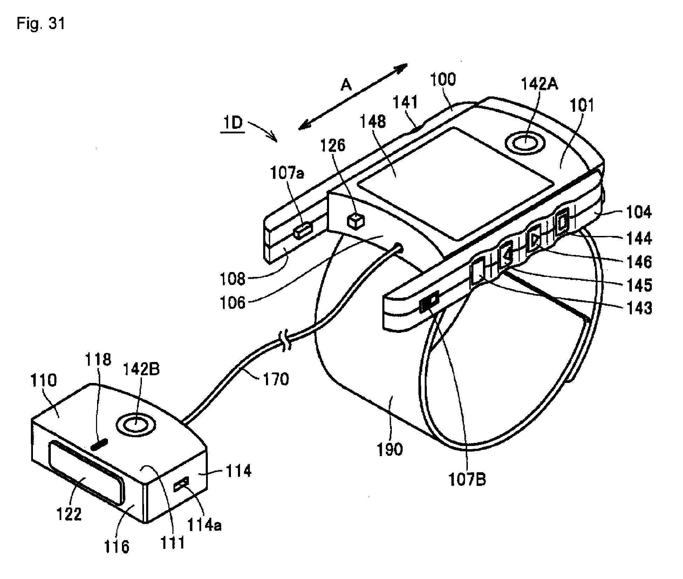

Fig. 29 is a perspective view showing a state where the detachable unit is united to the body unit in a portable electrocardiograph according to a fourth embodiment of the present invention. Fig. 30 is a rear view showing the state where the detachable unit is united to the body unit in the portable electrocardiograph according to the present embodiment. Fig. 31 is a perspective view showing a state where the detachable unit is separated from the body unit in the portable electrocardiograph according to the present embodiment. Same reference numerals are denoted for the portions similar to the first embodiment, and the description thereof will not be repeated herein.

As shown in Fig. 29 to Fig. 31, the portable electrocardiograph 1 D according to the present embodiment includes the body unit 100 serving as the first unit and the detachable unit 110 serving as the second unit, similar to the portable electrocardiograph 1A according to the first embodiment, and also includes a band 190 serving as an attachment mechanism to the living body. The band 190 is a wrapping member to be wrapped around the right hand of the examinee, and is fixed to the body unit 100 by being inserted to openings 102a to 102d formed at the rear face 102 of the body unit 100 as shown in Fig. 30. As shown in Fig. 31, the detachable unit 110 is detachably attached to the body unit 100.

The body unit 100 and the detachable unit 110 of the portable electrocardiograph 1 D according to the present embodiment substantially have the same configuration as the body unit 100 and the detachable unit 110 of the portable electrocardiograph 1A according to the first embodiment described above. That is, the display unit 148 and the first measurement button 142A are arranged on the front face 101 of the body unit 100 and various operation buttons 141, 143 to 146, the open/close cover 130, and the release buttons 107A, 107B are arranged on the upper face 103 and the lower face 104 of the body unit 100. The accommodating section 108 for receiving the detachable unit 110 is arranged on the left side face 106 side of the body unit 100. The second measurement button 142B is arranged on the front face 111 of the detachable unit 110, and the positive electrode 122 serving as the second electrode is arranged on the left side face 116 of the detachable unit 110.

In the portable electrocardiograph 1A according to the first embodiment, the negative electrode 121 serving as the first electrode and the indifferent electrode 124 are arranged on the right side face 105 of the body unit 100, but in the portable electrocardiograph 1 D according to the present embodiment, the negative electrode 121 serving as the first electrode and the indifferent electrode 124 are arranged on the rear face 102 of the body unit 100 as shown in Fig. 30. An opening 191 is formed in the band 190 at a position corresponding to the negative electrode 121 and the indifferent electrode 124 arranged on the rear face 102 of the body unit 100, and the negative electrode 121 and the indifferent electrode 124 are always maintained to be exposed.

According to such a configuration, since the examinee can constantly wear the portable electrocardiograph at the wrist of the right hand, the portable electrocardiograph excelling in portability can be obtained. Furthermore, since the negative electrode 121 and the indifferent electrode 124 are constantly in contact with the wrist of the right hand of the examinee through the opening 191 of the band 190, the detachable unit 110 can be detached from the body unit 100 and the measuring position can be quickly taken when a certain symptom to be measured occurs. The portable electrocardiograph excelling in handleability is thereby achieved.

The present invention has been specifically described based on the embodiments of the present invention, but the present invention should not be construed as being limited to each embodiment disclosed herein. The technical scope of the invention is defined by the appended claims, and all changes that fall within meets and bounds of the claims, or equivalence of such meets and bounds are therefore intended to be embraced by the claims.

## Claims

1. A portable electrocardiograph, comprising a first unit arranged with a first electrode and a second unit arranged with a second electrode, the electrocardiograph for obtaining an electrocardiographic waveform by measuring a potential difference generated between the first and the second electrodes brought into contact with a body surface; wherein
the second unit is detachably attached to the first unit,
the first electrode and the second electrode are both exposed in a state where the second unit is united to the first unit, and
a measurement of the potential difference for obtaining the electrocardiographic waveform is made between the first and the second electrodes both in the state where the second unit is united to the first unit and in a state where the second unit is separated from the first unit.

2. The portable electrocardiograph according to claim 1, wherein a measurement button for starting a measurement of the electrocardiographic waveform is arranged on both the first unit and the second unit.

3. The portable electrocardiograph according to claim 1, wherein
the first unit and the second unit are connected by a connection cable, and
a winding mechanism for winding and accommodating the connection cable is arranged either in the first unit or in the second unit.

4. The portable electrocardiograph according to claim 1, wherein
a device body including the first unit and the second unit has a substantially rectangular solid shape in the state where the second unit is united to the first unit, and
the second electrode is arranged on one end face in a longitudinal direction of the device body.

5. The portable electrocardiograph according to claim 1, wherein
the first electrode is to be brought into contact with a right hand, and
the second electrode is to be brought into contact with a chest region.

6. The portable electrocardiograph according to claim 5, wherein a display unit for displaying a measurement result is arranged in the first unit.

7. The portable electrocardiograph according to claim 5, wherein an attachment mechanism enabling attachment to a living body is arranged in the first unit.

8. The portable electrocardiograph according to claim 7, wherein the attachment mechanism includes a wrapping member to be wrapped around a wrist.

9. A portable electrocardiograph, comprising a first unit arranged with a first electrode, and a second unit arranged with a second electrode and a third electrode, the electrocardiograph for obtaining an electrocardiographic waveform by measuring a potential difference generated between the first and the second electrodes brought into contact with a body surface or between the third and the second electrodes brought into contact with the body surface; wherein
the second unit is detachably attached to the first unit,
the first electrode and the second electrode are exposed and the third electrode is covered by the first unit in a state where the second unit is united to the first unit,
the first electrode, the second electrode, and the third electrode are all exposed in a state where the second unit is separated from the first unit, and
a measurement of the potential difference for obtaining the electrocardiographic waveform is made between the first and the second electrodes in the state where the second unit is united to the first unit, and is made between the third and the second electrodes in the state where the second unit is separated from the first unit.

10. The portable electrocardiograph according to claim 9, further comprising a switching unit for switching between the first electrode and the third electrode to be used to obtain the electrocardiographic waveform.

11. The portable electrocardiograph according to claim 10, wherein the switching unit performs a switching operation in conjunction with at least one of an operation of separating the second unit from the first unit and an operation of uniting the second unit to the first unit.

12. The portable electrocardiograph according to claim 9, wherein a measurement button for starting a measurement of the electrocardiographic waveform is arranged on both the first unit and the second unit.

13. The portable electrocardiograph according to claim 9, wherein
the first unit and the second unit are connected by a connection cable, and
a winding mechanism for winding and accommodating the connection cable is arranged either in the first unit or in the second unit.

14. The portable electrocardiograph according to claim 9, wherein
a device body including the first unit and the second unit has a substantially rectangular solid shape in the state where the second unit is united to the first unit, and
the second electrode is arranged on one end face in a longitudinal direction of the device body.

15. The portable electrocardiograph according to claim 9, wherein
the first electrode is to be brought into contact with a right hand, and
the second electrode is to be brought into contact with a chest region.

16. The portable electrocardiograph according to claim 15, wherein a display unit for displaying a measurement result is arranged in the first unit.

17. The portable electrocardiograph according to claim 15, wherein an attachment mechanism enabling attachment to a living body is arranged in the first unit.

18. The portable electrocardiograph according to claim 17, wherein the attachment mechanism includes a wrapping member to be wrapped around a wrist.
